# EUROPEAN PATENT SPECIFICATION

(11) **EP 4 324 809 B1**
(45) Date of publication and mention of the grant of the patent: **09.07.2025**
(21) Application number: 23165973.1
(22) Date of filing: 31.03.2023
(51) Int. Cl.: C07B 59/00, C07C 273/02

(54) **CONTINUOUS-FLOW SYNTHESIS METHOD OF 13C-UREA**
VERFAHREN ZUR KONTINUIERLICHEN SYNTHESE VON 13C-HARNSTOFF
PROCÉDÉ DE SYNTHÈSE D'URÉE 13C À ÉCOULEMENT CONTINU

(30) Priority: 26.07.2022 CN 202210881735
(43) Date of publication of application: 21.02.2024
(73) Proprietor: Shenzhen Zhonghe Headway Bio-Sci & Tech Co., Ltd., Shenzhen Guangdong 518122 (CN); Harbin Institute Of Technology, Shenzhen, Shenzhen, Guangdong 518055 (CN)
(72) Inventor: QING, Jing, Shenzhen, 518122 (CN); YOU, Hengzhi, Shenzhen, 518055 (CN); CHEN, Kai, Shenzhen, 518122 (CN); HAN, Quansheng, Shenzhen, 518122 (CN); LI, Guowei, Shenzhen, 518122 (CN); LIAO, Jingyuan, Shenzhen, 518055 (CN); JIA, Xuelei, Shenzhen, 518122 (CN); DAI, Desheng, Shenzhen, 518122 (CN)
(74) Representative: Vitina, Maruta

(56) References cited:
- CN-A- 107 663 162
- CN-A- 107 663 163

## Description

### TECHNICAL FIELD

This application relates to urea synthesis, and more particularly to a continuous-flow synthesis method of ¹³C-urea.

### BACKGROUND

*Helicobacter pylori* (*H. pylori*), as a spiral-shaped bacterium with numerous unipolar flagella, was discovered in 1982 by Barry J. Marshall and J. Robin Warren. *H. pylori* has been proved to be associated with gastritis, chronic gastroenteritis, gastric ulcer, duodenal ulcer, non-ulcer dyspepsia and some gastric cancers, and thus has attracted a lot of medical attention. Extensive researches have been conducted to investigate the relationship between *H. pylori* infection and gastrointestinal diseases over the past 30 years.

The *H. pylori* is detected and identified mainly by rapid urease test, *H. pylori* antibody test, ¹³C-urea (or ¹⁴C-urea) breath test, pathological tissue section method, and culture, where the ¹³C-urea (or ¹⁴C-urea) breath test high the highest precision (95-96% or more). ¹³C-urea is an essential raw material for the production of the ¹³C-urea breath test kit, but it is still difficult to achieve the industrial preparation of ¹³C-urea.

Generally, the urea is synthesized through the high-temperature and high-pressure reaction between carbon dioxide (CO₂) and ammonia (NH₃). Whereas, this synthesis route is not suitable for the ¹³C-urea preparation due to the poor CO₂ conversion.

Chinese Patent Publication Application CN 107663162 A discloses a ¹³C-urea synthesis method. The reactor cooled through liquid nitrogen is filled with weighed methanol and sulfur, followed by liquefied ¹³CO and NH₃. The reaction vessel is closed and the temperature of the reaction vessel is set to 80-120°C. The reactor is purged by helium to empty the air in the reactor.

Chinese Patent Publication Application CN 107663163 A discloses a ¹³C-urea synthesis method. The reaction kettle is purged with helium, and is charged with gas at ultralow temperature into the reaction kettle connected to the feed gas cylinder. The gas filing process can be operated continuously and automatically through a pressure difference between the reaction kettle and the fed gas cylinder. The liquid ¹³CO and NH₃ together with sulfur reacts in the reaction kettle.

### SUMMARY

In view of the defects in the prior art, the present disclosure provides a highly-efficient and safe continuous-flow synthesis method of ¹³C-urea. In this continuous-flow synthesis method route, ¹³CO, sulphur (S) and NH₃ are reacted in methanol in a continuous-flow reactor under heating and pressurizing conditions to continuously prepare ¹³C-urea, with 95-100% ¹³CO conversion rate, 90-95%¹³C-urea yield and 99% ¹³C-urea purity. Therefore, the synthesis method provided herein has excellent product quality and high yield.

Technical solutions of the present disclosure are described as follows.

This application provides a continuous-flow synthesis method of ¹³C-urea, comprising:
(S1) mixing sulphur and a methanol solution containing NH₃ in a feed kettle to obtain a slurry; or mixing ammonia gas (NH₃), sulphur and methanol in a feed kettle to obtain a slurry;
(S2) feeding the slurry into a mixing unit; and feeding ¹³CO into the mixing unit to obtain a three-phase mixture;
(S3) mixing the three-phase mixture in the mixing unit evenly; and feeding the three-phase mixture into a continuous-flow reactor for reaction to obtain a reaction product; and
(S4) feeding the reaction product into a gas-liquid separator for gas-liquid separation, and collecting a liquid phase; subjecting the liquid phase to purification to obtain the ¹³C-urea.

In some embodiments, a molar ratio of the sulphur to NH₃ to ¹³CO is (1-5):(2-50):1.

In some embodiments, the continuous-flow reactor is controlled to 0-5 MPa and 50-150°C.

In some embodiments, in step (S2), a flow rate of the slurry is 0.001-10 L/min; and a flow rate of the ¹³CO is 0.001-100 L/min.

In some embodiments, a residence time of the three-phase mixture in the continuous-flow reactor is 1-120 min.

In some embodiments, in step (S4), the purification is performed through steps of:
subjecting the liquid phase to rotary evaporation in a rotary evaporator, dissolving with an alcohol or water, and vacuum filtration to collect a filtrate; and
drying the filtrate to obtain the ¹³C-urea.

In some embodiments, a purity of the ¹³C-urea after purification is 99%.

In some embodiments, a ¹³CO conversion rate is 95-100%; and a ¹³C-urea yield is 90-95%.

In some embodiments, a heat exchange medium of the continuous-flow reactor is heat-conducting oil or a water-based heat-conducting medium; and the water-based heat-conducting medium is water, a 1,2-ethanediol binary aqueous solution or a 1,2-propanediol binary aqueous solution.

In some embodiments, the slurry is fed by a feed pump into the mixing unit; the ¹³CO passes through a pressure reducing valve to enter the mixing unit, wherein a flow of the ¹³CO is controlled by a flow controller; and the reaction product flowing out from the continuous-flow reactor enters the gas-liquid separator through a back pressure valve.

Compared to the prior art, this application has the following beneficial effects.
1. This application additionally provides the mixing unit before the continuous-flow reactor to allow even mixing of the reaction materials, which can prevent the pipeline blockage caused by sedimentation, realizing the continuous ¹³C-urea synthesis. The continuous-flow synthesis method strategy provided herein has novel process, simple operation, high product quality and desirable yield, reducing the cost. Moreover, this application has simple purification, relatively mild reaction conditions, and low pollution. Therefore, the continuous-flow synthesis method provided herein is suitable for the industrial production of ¹³C-urea.
2. Compared to the batch reactor, the continuous-flow reactor enables the continuous-flow synthesis method, as well as the accurate control of the residence time of the reactants, allowing for improved yield and safety.
3. Compared to the batch reactor, the continuous-flow reactor can bring a higher ¹³C-urea yield, and can realize the continuous production of ¹³C-urea.
4. By means of the continuous-flow reactor, the reaction time is shortened from several hours to a few minutes, significantly enhancing the reaction efficiency and facilitating the large-scale production of ¹³C-urea.
5. The continuous-flow synthesis method provided herein enhances the mass transfer and heat transfer, and can keep the reaction temperature constant, thereby avoiding temperature runaway, material ejection and the loss of control, and effectively avoiding the leakage of toxic gases such as ¹³CO and H₂S. Moreover, the continuous-flow reactor has a small internal volume, such that the toxic substances exist in a relatively low level, greatly improving the operability and safety.

### BRIEF DESCRIPTION OF THE DRAWINGS

The drawings needed in the description of the embodiments of the disclosure or the prior art will be briefly described below to explain technical solutions of the embodiments of the present disclosure or the prior art more clearly. Obviously, presented in the accompany drawings are merely some embodiments of the present disclosure, and other drawings can be obtained by those skilled in the art based on the drawings provided herein without paying creative effort.

This figure is a flow chart of a continuous-flow synthesis method of ¹³C-urea according to an embodiment of the present disclosure.

Implementation, features and advantages will be further illustrated below with reference to the accompany drawing and embodiments.

### DETAILED DESCRIPTION OF EMBODIMENTS

Technical solutions of the present disclosure will be clearly and completely described below with reference to the embodiments and accompanying drawings. Obviously, described below are merely some embodiments of this disclosure, and are not intended to limit the disclosure. Other embodiments obtained by those skilled in the art based on the embodiments provided herein without paying any creative effort should fall within the scope of the present disclosure.

Unless otherwise specified, the materials and reagents in the following embodiments are available commercially, and the experiments are performed by conventional methods. For the quantitative analysis, three replicates are performed, and the results are expressed as mean or mean ± standard deviation.

As used herein, the "and/or" includes three solutions, for example, "A and/or B" includes A, B and a combination thereof. Additionally, technical solutions of various embodiments can be combined on the premise that the combined technical solution can be implemented by those skilled in the art. When the combination of technical solutions is contradictory or cannot be implemented, such a combination does not exist, and does not fall within the scope of the present disclosure.

Described herein was a continuous-flow synthesis method of ¹³C-urea, including steps of:
(S1) mixing sulphur and a methanol solution containing NH₃ in a feed kettle to obtain a slurry; or mixing ammonia gas (NH₃), sulphur and methanol in a feed kettle to obtain a slurry;
(S2) feeding the slurry into a mixing unit; and feeding ¹³CO into the mixing unit to obtain a three-phase mixture;
(S3) mixing the three-phase mixture in the mixing unit evenly; and feeding the three-phase mixture into a continuous-flow reactor for reaction to obtain a reaction product; and
(S4) feeding the reaction product into a gas-liquid separator for gas-liquid separation, and collecting a liquid phase; subjecting the liquid phase to purification to obtain the ¹³C-urea.

By replacing a kettle reactor with the continuous-flow reactor, a reaction time of continuous-flow synthesis is reduced from hours to minutes, significantly developing the reaction rate. In addition, the raw materials react in the continuous-flow reactor, contributing to a full contact therebetween. Particularly, the continuous-flow synthesis method using the continuous-flow reactor does not require gas compression into the reactor as opposed to the traditional kettle reactor reaction, shorting a process time, and improving a reaction efficiency.

Since a gas-phase raw material in the continuous-flow synthesis method is ¹³CO, the pressure in the continuous-flow reactor keeps constant during reaction and will not affect the reaction. To the contrary, regarding the reaction in the kettle reactor, ¹³CO therein will be consumed constantly, therefore, it is difficult to maintain the initial pressure in the reactor at the later stage of the reaction, resulting in reaction rate decreases and affecting the reaction efficiency.

In an embodiment, a molar ratio of the sulphur to NH₃ to ¹³CO is (1-5):(2-50):1.

In an embodiment, the continuous-flow reactor is controlled to 0-5 MPa and 50-150°C.

In an embodiment, in step (S2), a flow rate of the slurry is 0.001-10 L/min; and a flow rate of the ¹³CO is 0.001-100 L/min.

In an embodiment, a residence time of the three-phase mixture in the continuous-flow reactor is 1-120 min.

By using the above-mentioned technology solutions, the reaction time will be greatly reduced compared with traditional kettle-reactor reaction.

In an embodiment, in step (S4), the purification is performed through the following steps.

The liquid phase is subjected to rotary evaporation in a rotary evaporator, dissolving with an alcohol or water, and vacuum filtration to collect a filtrate. Then the filtrate is dried to obtain the ¹³C-urea.

In an embodiment, a purity of the ¹³C-urea after purification is 99%.

By using the above-mentioned technology solutions, the product yield is high.

In an embodiment, a ¹³CO conversion rate is 95-100%; and a ¹³C-urea yield is 90-95%.

In an embodiment, a heat exchange medium of the continuous-flow reactor is heat-conducting oil or a water-based heat-conducting medium; and the water-based heat-conducting medium is water, a 1,2-ethanediol binary aqueous solution or a 1,2-propanediol binary aqueous solution.

In an embodiment, the slurry is fed by a feed pump into the mixing unit; the ¹³CO passes through a pressure reducing valve to enter the mixing unit, wherein a flow of the ¹³CO is controlled by a flow controller; and the reaction product flowing out from the continuous-flow reactor enters the gas-liquid separator through a back pressure valve.

By using the above-mentioned technology solutions, the flow of the slurry and that of ¹³CO can be precisely controlled, thereby precisely controlling a reaction process.

In an embodiment, the continuous-flow synthesis method further includes a step of gas leak detection. The ¹³C-urea is synthesized in an operating room, which is kept in negative pressure by using an evacuating device. The evacuating device is communicated with an evacuating pipe. The operating room and the evacuating pipe are respectively provided with an ammonia sensing probe, a carbon monoxide sensing probe, and a hydrogen sulfide sensing probe. When levels of ammonia and/or carbon monoxide and/or hydrogen sulfide exceed a preset value, it indicates a possible gas leak. Since ammonia, carbon monoxide and hydrogen sulfide are hazardous to the health of operators, the system will give an alarm or a corresponding valve is immediately closed. The operators need to monitor and repair the pipe before continuing with the urea synthesis process.

The above-mentioned technology solutions can protect the operator from being damaged ammonia, carbon monoxide and hydrogen sulfide.

In summary, by means of the continuous-flow reactor, the continuous-flow synthesis method provided herein has novelty, simple operation, high product quality and yield, thereby leading to low cost and less pollution. By means of the continuous-flow reactor, the cost is greatly reduced. This application has simple operation, relatively mild reaction conditions, and relatively low pollution. The continuous-flow synthesis method provided herein effectively overcomes the defects of existing reactions that cannot be produced on a larger scale, facilitating large-scale production and improving quality and yield of the ¹³C-urea.

### Example 1

(S1) 71.4 g of sulphur, 1200 mL of a methanol solution containing 7 mol/L of NH₃ and 1000 mL of methanol were fed into a feed kettle, and then mixed to obtain a slurry.

(S2) The slurry was fed by a feed pump into a mixing unit, to which ¹³CO was fed after passing through a pressure reducing valve, so as to obtain a three-phase mixture, where a flow rate of the ¹³CO was controlled at 0.1 L/min by a flow controller, and a flow rate of the slurry was 40 mL/min.

(S3) The three-phase mixture was mixed evenly in the mixing unit, fed into a continuous-flow reactor, and reacted at 130°C and 3 MPa for 20 min to obtain a reaction product.

(S4) The reaction product was cooled by a cooling coil in an ice-water bath, and then flowed out of the continuous-flow reactor as a brown liquid to enter a gas-liquid separator for separation. A liquid phase was collected as a crude product solution, and subjected to rotary evaporation in a rotary evaporator, dissolving with water and vacuum filtration to collect a filtrate. The filtrate was dried to obtain ¹³C-urea. The gas phase generated from the gas-liquid separation was discharged and absorbed with a NaOH solution.

Examples 2-5 were performed according to the steps of Example 1. The amounts of raw materials and the reaction parameters of Examples 2-5 were shown in Table 1.

**Table 1 Amounts of raw materials and reaction parameters of Examples 1-5**

| | Example 1 | Example 2 | Example 3 | Example 4 | Example 5 |
|---|---|---|---|---|---|
| Sulphur/g | 71.4 | 7.4 | 357 | 1071 | 3570 |
| Volume of the NH₃-containing methanol solution /mL | 1200 | 120 | 4000 | 12000 | 40000 |
| Concentration of NH₃ in the methanol solution/mol·L⁻¹ | 7 | 7 | 7 | 7 | 7 |
| Volume of ethanol solution/mL | 1000 | 100 | 21000 | 63000 | 210000 |
| Flow rate of slurry/mL·min⁻¹ | 40 | 4 | 200 | 600 | 2000 |
| Flow rate of ¹³CO/L·min⁻¹ | 0.1 | 0.01 | 1 | 3 | 15 |
| Reaction temperature/°C | 130 | 110 | 100 | 120 | 110 |
| Pressure/MPa | 3 | 1 | 1 | 1 | 1 |
| Residence time/min | 20 | 20 | 40 | 40 | 40 |

| | | | | | |
|---|---|---|---|---|---|
| ¹³C-urea yields and ¹³CO conversion rates of Examples 1-5 were shown in Table 2. | | | | | |

**Table 2 ¹³C-urea yields and ¹³CO conversion rates of Examples 1-5**

| | Example 1 | Example 2 | Example 3 | Example 4 | Example 5 |
|---|---|---|---|---|---|
| ¹³C-urea yield/% | 92.3 | 90.1 | 94.1 | 91.7 | 90.8 |
| ¹³CO conversion rate/% | 100 | 98 | 100 | 98 | 96 |

Comparative Examples 1-2 were performed basically according to the steps of Example 1.

Regarding Comparative Example 1, a slurry obtained in step (S1) and ¹³CO were directly fed into the continuous-flow reactor for reaction.

Regarding Comparative Example 2, it was free from addition of 1000 mL of methanol in step (S1).

¹³C-urea yields and ¹³CO conversion rates of Comparative Examples 1-2 are shown in Table 3.

**Table 3 ¹³C-urea yields and ¹³CO conversion rates of Comparative Examples 1-2**

| | Comparative Example 1 | Comparative Example 2 |
|---|---|---|
| ¹³C-urea yield/% | 79.6 | 89.4 |
| ¹³CO conversion rate/% | 87 | 92 |

Technical solutions of various embodiments can be combined on the premise that the combined technical solution can be implemented by those skilled in the art. When the combination of technical solutions is contradictory or cannot be implemented, such a combination does not exist, and does not fall within the scope of the present disclosure.

Described above are only some embodiments of the present disclosure, which are not intended to limit the disclosure. The scope and limitation of the invention is as defined in the appended claims.

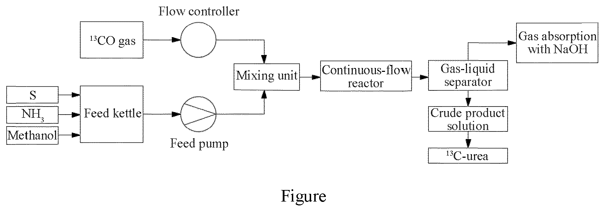

## Claims

1. A continuous-flow synthesis method of ¹³C-urea, comprising:
(S1) mixing sulphur and a methanol solution containing NH₃ in a feed kettle to obtain a slurry; or mixing ammonia gas (NH₃), sulphur and methanol in a feed kettle to obtain a slurry;
(S2) feeding the slurry into a mixing unit; and feeding ¹³CO into the mixing unit to obtain a three-phase mixture;
(S3) mixing the three-phase mixture in the mixing unit evenly; and feeding the three-phase mixture into a continuous-flow reactor for reaction to obtain a reaction product; and
(S4) feeding the reaction product into a gas-liquid separator for gas-liquid separation, and collecting a liquid phase; subjecting the liquid phase to purification to obtain the ¹³C-urea.

2. The continuous-flow synthesis method of claim 1, wherein a molar ratio of the sulphur to NH₃ to ¹³CO is (1-5):(2-50):1.

3. The continuous-flow synthesis method of claim 1, wherein the continuous-flow reactor is controlled to 0-5 MPa and 50-150°C.

4. The continuous-flow synthesis method of claim 1, wherein in step (S2), a flow rate of the slurry is 0.001-10 L/min; and a flow rate of the ¹³CO is 0.001-100 L/min.

5. The continuous-flow synthesis method of claim 1, wherein a residence time of the three-phase mixture in the continuous-flow reactor is 1-120 min.

6. The continuous-flow synthesis method of claim 1, wherein in step (S4), the purification is performed through steps of:
subjecting the liquid phase to rotary evaporation in a rotary evaporator, dissolving with an alcohol or water, and vacuum filtration to collect a filtrate; and
drying the filtrate to obtain the ¹³C-urea.

7. The continuous-flow synthesis method of claim 6, wherein a purity of the ¹³C-urea after purification is 99%.

8. The continuous-flow synthesis method of claim 1, wherein a ¹³CO conversion rate is 95-100%; and a ¹³C-urea yield is 90-95%.

9. The continuous-flow synthesis method of claim 1, wherein a heat exchange medium of the continuous-flow reactor is heat-conducting oil or a water-based heat-conducting medium; and the water-based heat-conducting medium is water, a 1,2-ethanediol binary aqueous solution or a 1,2-propanediol binary aqueous solution.

10. The continuous-flow synthesis method of claim 1, wherein the slurry is fed by a feed pump into the mixing unit; the ¹³CO passes through a pressure reducing valve to enter the mixing unit, wherein a flow of the ¹³CO is controlled by a flow controller; and the reaction product flowing out from the continuous-flow reactor enters the gas-liquid separator through a back pressure valve.

## Patentansprüche

1. Durchfluss-Syntheseverfahren für ¹³C -Harnstoff, umfassend:
(S1) Mischen von Schwefel und einer NH₃-haltigen Methanollösung in einem Beschickungskessel, um eine Aufschlämmung zu erhalten; oder Mischen von Ammoniakgas (NH₃), Schwefel und Methanol in einem Beschickungskessel, um eine Aufschlämmung zu erhalten;
(S2) Zuführen der Aufschlämmung in eine Mischeinheit; und Zuführen von ¹³CO in die Mischeinheit, um ein Dreiphasenmischung zu erhalten;
(S3) gleichmäßiges Mischen der Dreiphasenmischung in der Mischeinheit; Zuführen der Dreiphasenmischung in einen Durchflussreaktor zur Reaktion, um ein Reaktionsprodukt zu erhalten; und
(S4) Zuführen des Reaktionsprodukts in einen Gas-Flüssigkeits-Abscheider zur Gas-Flüssigkeits-Trennung und Sammeln einer Flüssigphase; Unterziehen der Flüssigphase einer Reinigung, um das ¹³C-Harnstoff zu erhalten.

2. Durchfluss-Syntheseverfahren nach Anspruch 1, wobei ein molares Verhältnis von Schwefel zu NH₃ zu ¹³CO (1-5):(2-50):1 beträgt.

3. Durchfluss-Syntheseverfahren nach Anspruch 1, wobei der Durchflussreaktor auf 0-5 MPa und 50-150°C geregelt wird.

4. Durchflussreaktor nach Anspruch 1, wobei in Schritt (S2) die Fließgeschwindigkeit der Aufschlämmung 0,001-10 L/min beträgt; und die Fließgeschwindigkeit des ¹³CO 0,001-100 L/min beträgt.

5. Durchfluss-Syntheseverfahren nach Anspruch 1, wobei eine Verweilzeit der Dreiphasenmischung im Durchflussreaktor 1-120 Minuten beträgt.

6. Durchfluss-Syntheseverfahren nach Anspruch 1, wobei in Schritt (S4) die Reinigung durch folgende Schritte durchgeführt wird:
Unterziehen der Flüssigphase einer Rotationsverdampfung in einem Rotationsverdampfer, Auflösen mit einem Alkohol oder Wasser und Vakuumfiltration, um ein Filtrat zu sammeln; und
Trocknen des Filtrats, um das ¹³C-Harnstoff zu erhalten.

7. Durchfluss-Syntheseverfahren nach Anspruch 6, wobei die Reinheit des ¹³C-Harnstoffs nach der Reinigung 99 % beträgt.

8. Durchfluss-Syntheseverfahren nach Anspruch 1, wobei die ¹³CO Umwandlungsrate 95-100 % beträgt; und die ¹³C-Harnstoffausbeute 90-95 % beträgt.

9. Durchfluss-Syntheseverfahren nach Anspruch 1, wobei ein Wärmeaustauschmedium des Durchflussreaktors wärmeleitendes Öl oder ein wasserbasiertes wärmeleitendes Medium ist; und wobei das wasserbasierte wärmeleitende Medium Wasser, eine 1,2-Ethandiol-binäre wässrige Lösung oder eine 1,2-Propandiolbinäre wässrige Lösung ist.

10. Durchfluss-Syntheseverfahren nach Anspruch 1, wobei die Aufschlämmung durch eine Förderpumpe in die Mischeinheit eingespeist wird; das ¹³CO durch ein Druckminderventil in die Mischeinheit eintritt, wobei ein Fluss des ¹³CO durch einen Durchflussregler gesteuert wird; und das aus dem Durchflussreaktor austretende Reaktionsprodukt durch ein Rückdruckventil in den Gas-Flüssig-Abscheider eintritt.

## Revendications

1. Procédé de synthèse en flux continu de ¹³C-urée, comprenant :
(S1) mélanger du soufre et une solution de méthanol contenant du NH₃ dans un réacteur d'alimentation pour obtenir une boue ; ou mélanger du gaz ammoniac (NH₃), du soufre et du méthanol dans un réacteur d'alimentation pour obtenir une boue ;
(S2) introduire la boue dans une unité de mélange ; et introduire du ¹³CO dans l'unité de mélange pour obtenir un mélange triphasé ;
(S3) mélanger uniformément le mélange triphasé dans l'unité de mélange ; et introduire le mélange triphasé dans un réacteur à flux continu pour réaction afin d'obtenir un produit de réaction ; et
(S4) introduire le produit de réaction dans un séparateur gaz-liquide pour la séparation gaz-liquide, et recueillir une phase liquide ; et soumettre la phase liquide à une purification pour obtenir de la ¹³C-urée.

2. Procédé de synthèse en flux continu selon la revendication 1, dans lequel un rapport molaire du soufre au NH₃ au ¹³CO est (1-5):(2-50): 1.

3. Procédé de synthèse en flux continu selon la revendication 1, dans lequel le réacteur à flux continu est contrôlé à 0-5 MPa et à 50-150°C.

4. Procédé de synthèse en flux continu selon la revendication 1, dans lequel, à l'étape (S2), un débit de la boue est de 0,001 à 10 L/min ; et un débit du ¹³CO est de 0,001 à 100 L/min.

5. Procédé de synthèse en flux continu selon la revendication 1, dans lequel un temps de séjour du mélange triphasé dans le réacteur à flux continu est de 1 à 120 min.

6. Procédé de synthèse en flux continu selon la revendication 1, dans lequel, à l'étape (S4), la purification est réalisée par les étapes suivantes :
soumettre la phase liquide à une évaporation rotative dans un évaporateur rotatif, dissoudre avec un alcool ou de l'eau et filtrer sous vide pour recueillir un filtrat ; et
sécher le filtrat pour obtenir la ¹³C-urée.

7. Procédé de synthèse en flux continu selon la revendication 6, dans lequel une pureté de la ¹³C-urée après purification est de 99 %.

8. Procédé de synthèse en flux continu selon la revendication 1, dans lequel un taux de conversion du ¹³CO est de 95 à 100 % et un rendement en ¹³C-urée est de 90 à 95 %.

9. Procédé de synthèse en flux continu selon la revendication 1, dans lequel un milieu d'échange de chaleur du réacteur à flux continu est une huile thermoconductrice ou un milieu thermoconducteur à base d'eau ; et le milieu thermoconducteur à base d'eau est de l'eau, une solution aqueuse binaire de 1,2-éthanediol ou une solution aqueuse binaire de 1,2-propanediol.

10. Procédé de synthèse en flux continu selon la revendication 1, dans lequel la boue est introduite par une pompe d'alimentation dans l'unité de mélange ; le ¹³CO traverse un détendeur de pression pour entrer dans l'unité de mélange, dans lequel un flux de ¹³CO est contrôlé par un contrôleur de flux ; et le produit de réaction s'écoulant du réacteur à flux continu entre dans le séparateur gaz-liquide par une vanne de contre-pression.
